# EUROPEAN PATENT APPLICATION

(11) **EP 3 663 278 A1**
(43) Date of publication of application: **10.06.2020**
(21) Application number: 18793743.8
(22) Date of filing: 24.04.2018
(51) Int. Cl.: C07C 211/25, C07C 209/48, C07C 211/31, C07C 253/22, C07C 253/30

(54) **DIAMINE COMPOUND, PRODUCTION METHOD FOR DIAMINE COMPOUND, AND POLYIMIDE**

(30) Priority: 01.05.2017 JP 2017091442
(71) Applicant: Arakawa Chemical Industries, Ltd., Osaka-shi Osaka 541-0046 (JP)
(72) Inventor: SATO, Yoshimasa, Tsukuba-shi Ibaraki 300-2611 (JP); TAKAHASHI, Kouji, Osaka-shi Osaka 538-0053 (JP); AOYAMA, Satoru, Tsukuba-shi Ibaraki 300-2611 (JP); KASHIHARA, Tetsuya, Tsukuba-shi Ibaraki 300-2611 (JP)
(74) Representative: KIPA AB
(86) International application number: PCT/JP2018/016672
(87) International publication number: WO 2018/203497

(57) **Abstract**

A diamine compound represented by H₂NH₂C-Ro²-CH₂NH₂, wherein Ro² represents a residue of a resin acid dimer.

## Description

### TECHNICAL FIELD

The present invention relates to a diamine compound, a method of preparing the diamine compound and polyimide. More specifically, the present invention relates to a diamine compound having a residue of a resin acid dimer as a main skeleton, a method of preparing the diamine compound and polyimide.

A diamine compound is a compound widely used in the fields of organic chemistry and polymer chemistry, and is useful as a monomer for a polyimide resin used particularly for a fiber, an electronic material, a material for a vehicle, a material for aerospace application or the like. It is widely known that a polyimide resin is excellent in heat resistance, mechanical properties and chemical resistance. As applications of a polyimide resin increase, higher heat resistance and mechanical properties are demanded compared to conventional polyimide. In particular, polyimide having a bulky skeleton is excellent in heat resistance (see Patent Document 1).

### PRIOR ART DOCUMENT

### Patent Document

Patent Document 1: JP H10-298150 A

### SUMMARY OF THE INVENTION

It is considered that a diamine compound having an alicyclic structure in its molecule and provided with a rigid and bulky skeleton not only provides a polymer having more excellent heat resistance but also develops unprecedented applications. Therefore, an object of the present invention is to provide a diamine compound having further excellent heat resistance and being capable of developing various new applications, a method of preparing a diamine compound and polyimide.

The diamine compound according to one embodiment of the present invention for solving the above-mentioned problem is a diamine compound represented by H₂NH₂C-Ro²-CH₂NH₂ (wherein Ro² represents a residue of a resin acid dimer).

A method of preparing a diamine compound according to one embodiment of the present invention for solving the above-mentioned problem is a method of preparing a diamine compound, comprising a step of preparing the diamine compound by subjecting a dinitrile compound represented by N≡C-Ro²-C≡N (wherein Ro² represents a residue of a resin acid dimer) to reduction reaction.

Polyimide according to one embodiment of the present invention for solving the above-mentioned problem is polyimide comprising a monomer unit derived from the above-mentioned diamine compound and a monomer unit derived from tetracarboxylic dianhydride.

### BRIEF DESCRIPTION OF DRAWINGS

FIG. 1 is an infrared absorption spectrum chart of dimerized rosin diamine obtained in Example 1.
FIG. 2 is a DI/MS chart of dimerized rosin diamine obtained in Example 1.
FIG. 3 is a GPC chart of dimerized rosin diamine obtained in Example 1.
FIG. 4 is an infrared absorption spectrum chart of dimerized rosin diamine obtained in Example 3.
FIG. 5 is a ¹HNMR chart of dimerized rosin diamine obtained in Example 3.
FIG. 6 is a GPC chart of dimerized rosin diamine obtained in Example 3.
FIG. 7 is an infrared absorption spectrum chart of polyimide obtained in Example 4.

### EMBODIMENT FOR CARRYING OUT THE INVENTION

The diamine compound according to one embodiment of the present invention is a compound represented by H₂NH₂C-Ro²-CH₂NH₂ (wherein Ro² represents a residue of a resin acid dimer, hereinafter, explanation of the formula will be omitted). One example of a structure thereof is shown below.

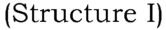

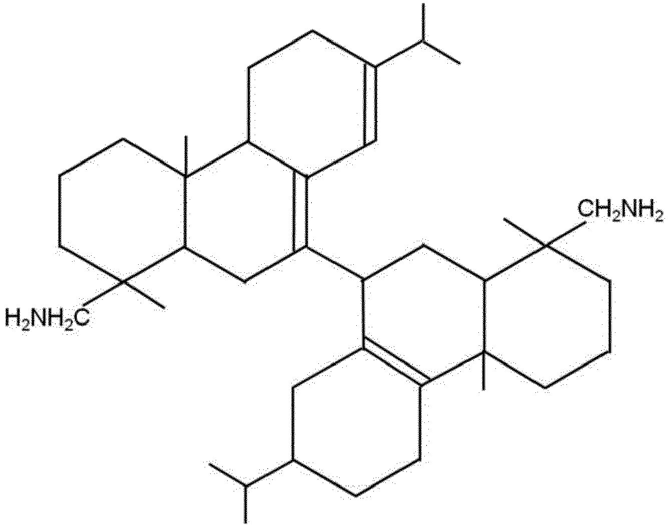

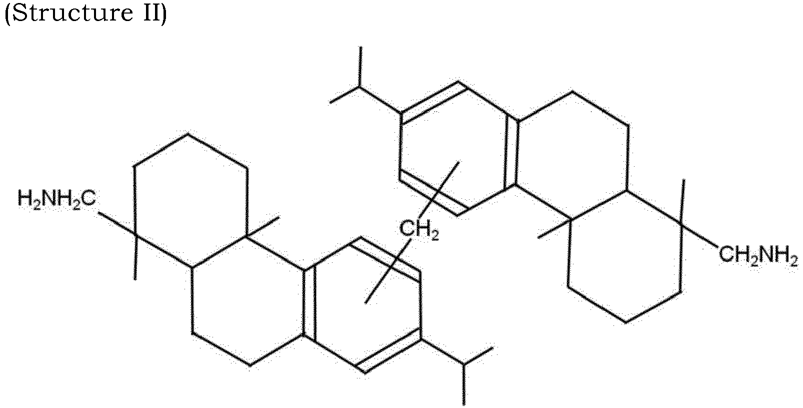

A resin acid dimer which is a precursor of the diamine compound according to the embodiment of the present invention is represented by HOOC-Ro²-COOH (wherein, Ro² represents a residue of a resin acid dimer. Hereinafter, explanation of the formula will be omitted). Herein, "a residue of resin acid dimer" refers to a portion remaining by excluding the two carboxyl groups from the resin acid dimer. Ro² is not particularly limited. Example of Ro² includes a structure represented by Ro¹-L₁-Ro¹ (wherein Ro¹ represents a residue of the resin acid and L₁ represents a single bond or a methylene group). Herein, "a residue of resin acid" refers to a portion remaining by excluding a carboxyl group from the resin acid represented by Ro¹-COOH. Examples of a structure of the resin acid dimer are shown below.

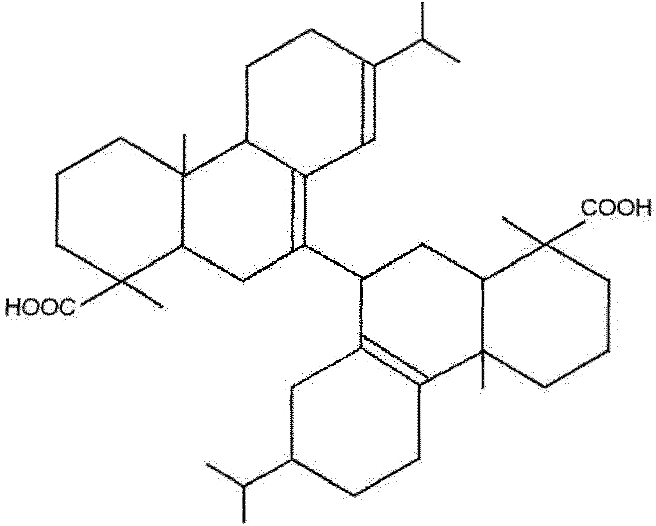

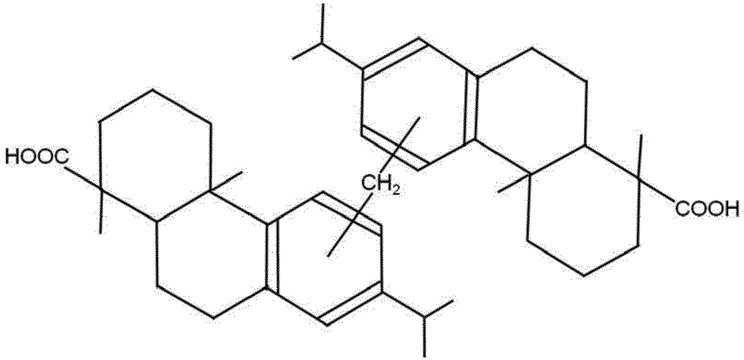

The diamine compound according to the embodiment of the present invention can be prepared by subjecting a dinitrile compound represented by N≡C-Ro²-C≡N (wherein Ro² represents a residue of a resin acid dimer. Hereinafter, explanation of the formula will be omitted) to reduction reaction.

The dinitrile compound can be prepared by a method of subjecting a mononitrile compound represented by Ro¹-C≡N, (wherein Ro¹ represents a residue of a resin acid. Hereinafter, explanation of the formula will be omitted) to dimerization reaction (hereinafter also referred to as a method [1]), or a method of allowing a diester compound represented by ROOC-Ro²-COOR, (wherein Ro² represents a residue of a resin acid dimer and R represents an alkyl group having 1 to 10 carbon atoms. Hereinafter, explanation of the formula will be omitted) to react with ammonia (hereinafter also referred to as a method [2]).

### <Method [1]>

The mono-nitrile compound being a starting material of the method [1] can be obtained by subjecting a resin acid and ammonia to nitrilation reaction by any of various known methods.

Examples of the resin acids include abietic acid, neoabietic acid, palustric acid, levopimaric acid, dehydroabietic acid, dihydroabietic acid, tetrahydroabietic acid, pimaric acid, isopimaric acid, sandaracopimaric acid, dehydroabietic acid and the like.

Resin acids are contained in various rosins. Specifically, examples of various rosins include natural rosins (gum rosin, tall oil rosin, wood rosin) derived from horsetail pine, slash pine (swamp pine), Merkus pine, Khasi pine, loblolly pine, longleaf pine and the like and purified rosins obtained by purification of natural rosins, hydrogenated rosins obtained by hydrogenation of natural rosins, disproportionated rosin obtained by subjecting natural rosin to disproportionation reaction.

The method and conditions for the above nitrilation reaction are not particularly limited. The method and conditions of the nitrilation reaction can employ a variety of known means. Specifically, for the nitrilation reaction, there is a method of heating and melting the rosin, and blowing ammonia gas to a molten product by various known means. Blowing conditions are not particularly limited. Usually, blowing conditions are such that an amount of ammonia is about 0.5 moles to about 20 moles per hour with respect to 1 mole of a resin acid contained in the rosin, a reaction temperature is from about 140°C to 360°C, and a reaction time is from about 1 hour to about 50 hours. At the time of the nitrilation reaction, a catalyst, for example, zinc oxide, calcium hydroxide, magnesium hydroxide or the like can be used. An amount of catalyst is not particularly limited. The amount of catalyst is usually from about 0.1 part by weight to about 50 parts by weight, preferably from about 1 part by weight to about 20 parts by weight based on 100 parts by weight of rosin.

An amount of mononitrile compound represented by Ro¹-C≡N contained in the product (rosin nitrile) obtained above is not particularly limited. The amount of mononitrile compound is usually 80 wt% or more, preferably 85 wt% or more, further preferably 90 wt% or more, and is about less than 99.9 wt%. Further, the product may be purified by various known means. The content of mononitrile compound may be determined by various known means. Specifically, the content of mononitrile compound is obtained from a ratio of a peak area corresponding to the mononitrile compound to a sum of areas of the total peaks of rosin nitrile using a GC/MS analysis method.

Then, the mononitrile compound is subjected to dimerization reaction. The dimerization reaction is not particularly limited. A variety of known methods can be employed for the dimerization reaction. Specifically, examples of the dimerization reaction include a method of heating the above-mentioned rosin nitrile in the presence or absence of a catalyst and an organic solvent (hereinafter referred to as a method [1]-1), or a method of reacting the rosin nitrile in the presence of formaldehyde, an acid catalyst and an organic solvent (hereinafter referred to as a method [1]-2).

Examples of the catalysts for the method [1]-1 include acid catalysts such as sulfuric acid, formic acid, acetic acid, phosphoric acid, p-toluenesulfonic acid, methanesulfonic acid and solid acids having a sulfonic acid group; Lewis acid catalysts such as hydrogen fluoride, zinc chloride, aluminum chloride, titanium tetrachloride, and boron trifluoride derivatives such as boron trifluoride and phenol boron trifluoride complex, boron trifluoride-dimethyl ether complex or boron trifluoride-diethyl ether complex; pendant sulfonic acid group-containing polymers such as polystyrene sulfonic acid, polyvinyl sulfonic acid and sulfonic acid functional group-containing fluorine-containing polymer and the like. The catalysts for the method [1]-1 may be used in combination. From the viewpoint of ease of removal, at least one selected from the group consisting of sulfuric acid, formic acid, phosphoric acid, p-toluenesulfonic acid, methanesulfonic acid, solid acids having a sulfonic acid group and zinc chloride is particularly preferred. An amount of catalyst is not particularly limited. The amount of catalyst is usually from about 0.1 part by weight to about 90 parts by weight, preferably from about 1 part by weight to about 20 parts by weight based on 100 parts by weight of the rosin nitrile. Further, heating conditions are not particularly limited in the above method. The heating conditions are usually from 0°C to about 200°C and from about 0.5 hour to about 24 hours.

Examples of the organic solvent for the method [1]-1 include aromatic hydrocarbons such as toluene, xylene and tetralin: aliphatic hydrocarbons such as heptane and octane: ketone-based hydrocarbons such as methyl ethyl ketone and methyl isopropyl ketone: ester-based hydrocarbons such as ethyl acetate and butyl acetate: halogenated hydrocarbons such as carbon tetrachloride, ethylene dichloride, trichloroethane and tetra-trichloroethane: carboxyl group-containing organic acids such as acetic acid, propionic acid, butyric acid and anhydrides thereof, formic acid, chloroacetic acid and lactic acid; and the like. The organic solvents for the method [1]-1 may be used in combination. In the case of using formic acid and acetic acid, because they also act as a polymerization catalyst, the above-mentioned catalysts may not be used. From the viewpoint of ease of recovery and reuse, the aromatic hydrocarbon and/or the aliphatic hydrocarbons are preferred as the organic solvent for the method [1]-1, and xylene, heptane and octane are particularly preferred. An amount of organic solvent is not particularly limited. The amount of organic solvent is usually from about 1 part by weight to about 900 parts by weight, preferably from about 1 part by weight to about 500 parts by weight based on 100 parts by weight of the rosin nitrile.

The acid catalysts for the method [1]-2 are, for example, the acid catalysts for the method [1]-1 and the like. The acid catalysts for the method [1]-2 may be used in combination. An amount of catalyst is not particularly limited. The amount of catalyst is usually from about 0.1 part by weight to about 200 parts by weight, preferably from about 1 part by weight to about 50 parts by weight based on 100 parts by weight of the rosin nitrile. Further, reaction conditions in the method [1]-2 are not particularly limited. The reaction conditions are usually from about -30°C to about 200°C, preferably from about 0°C to about 100°C, and from about 0.5 hour to about 24 hours.

The organic solvents for the method [1]-1 can be exemplified as the organic solvent for the method [1]-2. The organic solvents for the method [1]-2 may be used in combination. From the viewpoint of high solubility of the starting material, the organic solvent for the method [1]-2 are preferably aromatic hydrocarbons and/or ester-based hydrocarbons, particularly preferably ethyl acetate, butyl acetate and the like. An amount of organic solvent is not particularly limited. The amount of organic solvent is usually from about 1 part by weight to about 500 parts by weight, preferably from about 1 part by weight to about 200 parts by weight based on 100 parts by weight of the rosin nitrile.

The formaldehyde of the method [1]-2 is not particularly limited. Examples of the formaldehyde of the method [1]-2 include paraformaldehyde, formalin and the like. The formaldehyde of the method [1]-2 may be used in combination. From the viewpoint of solubility, paraformaldehyde is suitable as the formaldehyde of the method [1]-2. An amount of formaldehyde is not particularly limited. The amount of formaldehyde is usually from about 1 part by weight to about 100 parts by weight, preferably from about 1 part by weight to about 30 parts by weight based on 100 parts by weight of the rosin nitrile.

After completion of the dimerization reactions of the methods [1]-1 and [1]-2, a dinitrile compound represented by N≡C-Ro²-C≡N is obtained by removing the above-mentioned solvent, the catalyst, the un-reacted mononitrile compound, a decomposition product and the like from the product (dimerized rosin dinitrile) by washing with water, alkali neutralization, filtration, distillation under reduced pressure or the like if needed. Conditions for the distillation under reduced pressure is not particularly limited. The conditions for the distillation under reduced pressure is usually from about 200°C to about 290°C and from about 60 Pa to about 8,000 Pa. A content of the dinitrile compound in the dimerized rosin dinitrile is not particularly limited. The content of the dinitrile compound is usually 80 wt% or more, preferably 85 wt% or more, further preferably 90 wt% or more and is less than 99.9 wt%. The content of dinitrile compound can be determined by various known means. Specifically, the content of dinitrile compound is obtained by a method using a GC/MS analysis method mentioned above.

### <Method [2]>

One example of a structure of the diester compound represented by ROOC-Ro²-COOR which is a starting material for the method [2] is shown below. 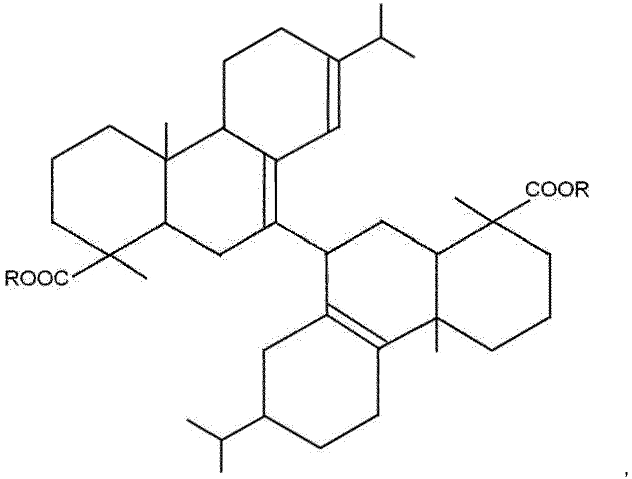 wherein R represents an alkyl group having 1 to 10 carbon atoms. 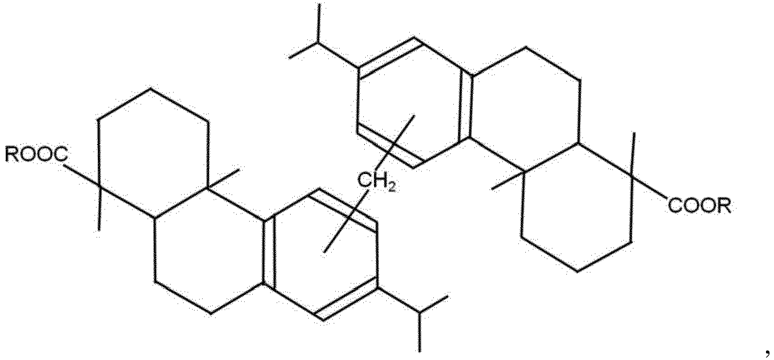 wherein R represents an alkyl group having 1 to 10 carbon atoms.

The diester compounds can be prepared by various known methods. Specifically, for example, a method described in WO 2014/030652 is exemplified as the method for preparing the diester compound.

The method for preparing the diester compound is not particularly limited. The diester compound can be prepared by various known methods. Specifically, examples of the method for preparing the diester compound include a method of subjecting the rosin or its chloride to esterification reaction with a monoalcohol having approximately 1 to 10 carbon atoms and dimerizing an obtained rosin ester (hereinafter referred to as a method [2]-1), or a method of subjecting a dimerized rosin obtained by dimerization reaction to esterification reaction with a monoalcohol (hereinafter referred to as a method [2]-2).

Examples of the monoalcohol include methanol, ethanol, propanol, isopropanol, butanol, i-butanol, t-butyl alcohol, pentanol and the like. From the viewpoint of yield of the diester compound, monoalcohols having 1 to about 5 carbon atoms are preferred, and methanol is particularly preferred.

The esterification reaction of the method [2]-1 is not particularly limited. Various known methods can be employed for the esterification reaction of the method [2]-1. Specifically, there is a method of an esterification reaction of the above-mentioned rosin with the above-mentioned monoalcohol. Specifically, examples of the esterification reaction of the method [2]-1 include a method of forming a resin acid contained in the above-mentioned rosin into, for example, resin acid chloride by a thionyl chloride method and then reacting the obtained resin acid chloride with the above-mentioned monoalcohol, and a method of reacting a resin acid with the above-mentioned monoalcohol under pressure and then removing a mixed solution of monoalcohol and water from a reaction system and further charging a new monoalcohol to perform an esterification reaction again.

The rosin ester obtained by the method [2]-1 comprises a monoester compound represented by Ro¹-COOR, wherein Ro¹ is a resin acid residue, R represents an alkyl group having 1 to 10 carbon atoms. Hereinafter, explanation of the formula will be omitted.

For the dimerization reaction of the monoester of the method [2]-1, a similar method and similar conditions for the dimerization reaction of the mononitrile compound described above can be adopted. After completion of the dimerization reaction, if necessary, an organic solvent, a catalyst, unreacted monoesters and a decomposition product can be removed from the product by the above-mentioned means and conditions.

The product (dimerized rosin diester) obtained in the method [2]-1 comprises a diester compound represented by ROOC-Ro²-COOR. An amount of the diester compound is not particularly limited. A content of diester compound is usually 80 wt% or more, preferably 85 wt% or more, further preferably 90 wt% or more and is less than 99.9 wt%. The content of diester compounds can be determined by various known means. Specifically, the content of diester compound is obtained from a ratio of a peak area corresponding to the diester compound to a sum of areas of total peaks of dimerized rosin diester by using a gel permeation chromatography (GPC) measurement.

For the dimerization reaction of the rosin in the method [2]-2, the dimerization reaction and its conditions described above can be employed.

The product (dimerized rosin) obtained in the method [2]-2 comprises a resin acid dimer represented by HOOC-Ro²-COOH. A content of resin acid dimer is not particularly limited. The content of resin acid dimer is usually 80 wt% or more, preferably 85 wt% or more, further preferably 90 wt% or more, and is less than 99.9 wt%. The content of resin acid dimer can be determined by various known means. Specifically, the content of resin acid dimer is obtained by a method using a gel permeation chromatography (GPC) measurement as described above.

For the esterification reaction of the dimerized rosin and the monoalcohol in the method [2]-2, the above-mentioned esterification method and conditions thereof can be employed. Specifically, examples of the esterification reaction include a method of subjecting a dimerized rosin to, for example, a thionyl chloride method to obtain acid chloride and then reacting the obtained acid chloride with the above-mentioned monoalcohol, and a method of reacting a dimerized rosin with the above-mentioned monoalcohol under pressure and then removing a mixed solution of monoalcohol and water from a reaction system and further charging a new monoalcohol to perform an esterification reaction again.

The product (dimerized rosin diester) obtained by the method [2]-2 comprises a diester compound represented by ROOC-Ro²-COOR. A content of diester compound is not particularly limited. The content of diester compound is usually 80 wt% or more, preferably 85 wt% or more, further preferably 90 wt% or more, and is less than 99.9 wt%. The content of diester compound can be determined by various known means. Specifically, the content of the diester compound is obtained by a method using the gel permeation chromatography (GPC) measurement as described above.

The same method and conditions as in the above nitrilation reaction described above can be employed as a method of reacting ammonia with the dimerized rosin diester obtained by the method [2]-1 and [2]-2. Specifically, example of the nitrilation reaction include a method of heating and melting the dimerized rosin diester, and blowing ammonia gas to the molten product under the above-mentioned conditions. Further, the catalyst for nitrilation reaction described above can be used in the reaction.

After completion of the nitrilation reaction, by removing the catalyst, the unreacted mononitrile compound, the decomposed product, etc. from the product (dimerized rosin dinitrile) as needed by washing with water, alkali neutralization, filtration, distillation under reduced pressure or the like, a dinitrile compound represented by N≡C-Ro²-C≡N is obtained. A content of dinitrile compound in the dimerized rosin dinitrile is not particularly limited. The content of dinitrile compound is usually 80 wt% or more, preferably 85 wt% or more, further preferably 90 wt% or more and is less than 99.9 wt%.

In the dimerized rosin dinitrile, for identification and determination of the dinitrile compound represented by N≡C-Ro²-C≡N, various known means can be employed. Specifically, examples of the identification and determination means for dinitrile compounds include an infrared spectroscopy method, a nuclear magnetic resonance method, a gas chromatography (GC) analysis, a GC/MS method and the like.

The diamine compound according to one embodiment of the present invention can be prepared by subjecting the dinitrile compounds obtained in the above methods [1] and [2] to reduction reaction. The method and conditions for the reduction reaction are not particularly limited. For the reduction reaction, a variety of known methods and conditions can be employed. Specifically, examples of the method and conditions for the reduction reaction include a method of subjecting a dinitrile compound to reduction in the presence of a reducing reagent and a solvent (hydride reduction), or a method of reducing the dinitrile compound by heating in the presence of a hydrogenation catalyst under hydrogen pressure (hydrogenation).

The method and conditions for the hydride reduction are not particularly limited. For the hydride reduction, various known methods and conditions can be employed. The reducing reagent is not particularly limited. Examples of the reducing reagent include boronhydride-based reducing reagent such as lithium boronhydride, sodium tetrahydroborate, sodium cyanotrihydroborate, lithium triethylhydroborate, sodium triacetoxyboron hydride and lithium tri(sec-butyl)boron hydride; aluminum hydride reducing reagent such as lithium aluminum hydride, diisobutylaluminum hydride, lithium triethoxy aluminum hydride and sodium bis(2-methoxyethoxy) aluminum hydride (SMEAH). An amount of reducing reagent is not particularly limited. The amount of reducing reagent is usually from about 0.1 part by weight to about 500 parts by weight, preferably from about 1 part by weight to about 300 parts by weight based on 100 parts by weight of the dinitrile. The above-mentioned solvent is not particularly limited. Examples of the solvent include aromatic hydrocarbons such as toluene, xylene and tetralin; aliphatic hydrocarbons such as heptane and octane; ketone-based hydrocarbons such as methyl ethyl ketone and methyl isopropyl ketone; ester-based hydrocarbons such as ethyl acetate and butyl acetate: halogenated hydrocarbons such as carbon tetrachloride, ethylene dichloride, trichloroethane and tetra-trichloroethane; and the like. These solvents may be used in combination. An amount of solvent is not particularly limited. The amount of solvent is usually from about 1 part by weight to about 800 parts by weight, preferably from about 1 part by weight to about 500 parts by weight based on 100 parts by weight of the dinitrile compound. Further, a reaction temperature also is not particularly limited. The reaction temperature is from about -30°C to about 200°C, and a reaction time is from about 0.5 hour to about 24 hours.

A method and conditions of hydrogenation are not particularly limited. Various known method and conditions for the hydrogenation can be employed. The hydrogenation is carried out, for example, by heating the dinitrile in the presence of a hydrogenation catalyst usually under pressurized hydrogen condition of from about 1 MPa to about 100 MPa, preferably from about 1 MPa to about 20 MPa for about 0.5 hour to about 24 hours. The hydrogenation catalyst is not particularly limited, and various known catalysts may be used. Examples of preferred hydrogenation catalyst include nickel-, cobalt-, palladium-, rhodium-, ruthenium- and platinum-based catalysts. The hydrogenation catalyst is usually used supported on a carrier such as carbon, silica or alumina. An amount of hydrogenation catalyst is usually from about 0.1 part by weight to about 100 parts by weight, preferably from about 0.1 part by weight to about 40 parts by weight based on 100 parts by weight of the dinitrile compound. Further, a hydrogenation temperature is from 0°C to about 300°C, preferably from about 10°C to about 200°C. Moreover, the hydrogenation, if desired, may be carried out in a state of the dinitrile compound being dissolved in a solvent. The solvent to be used is not particularly limited. Solvent is preferably one which is a solvent being inactive in the reaction and easily dissolves a raw material or a product. Examples of the solvents include cyclohexane, n-hexane, n-heptane, decalin, tetrahydrofuran, dioxane and the like. Solvents may be used in combination. An amount of solvent is not particularly limited. The solvent is usually used so that a solid content would be about 10% by weight or more relative to the dinitrile compound. The solid content is preferably within a range of from 10 to 70 wt%. In order to suppress by-production of secondary amines and tertiary amines, ammonia, an alkaline aqueous solution, a solid base and the like may be added to the solvent. An adding amount is not particularly limited. The adding amount is preferably 1 to 60 parts by weight based on 100 parts by weight of the dinitrile compound.

The diamine compound according to one embodiment of the present invention can be obtained even by subjecting the dimerized rosin dinitrile obtained in the methods [1] and [2] in place of the above-mentioned dinitrile compound to reduction reaction. For the reduction reaction of dimerized rosin dinitrile, the above-mentioned hydride reduction or hydrogenation can be employed by the similar method and conditions.

After completion of the reduction reaction, a diamine compound represented by H₂NH₂C-Ro²-CH₂NH₂ is obtained by removing the catalyst, an unreacted monoamine compound, a decomposed product, etc. from the product (dimerized rosin diamine) as needed by means of washing with water, alkali neutralization, filtration or distillation under reduced pressure. A content of diamine compound in the dimerized rosin diamine is not particularly limited. The content of diamine compound is usually 60 wt% or more, preferably 80 wt% or more, further preferably 99 wt% or more.

In the dimerized rosin diamine according to the embodiment of the present invention, for identification and determination of the diamine compound represented by H₂NH₂C-Ro²-CH₂NH₂, various known means may be employed. Specifically, examples of the identification and determination means for the diamine compound include an infrared spectroscopy method, a nuclear magnetic resonance method, a gel permeation chromatography (GPC), a gas chromatography (GC) analysis, a DI/MS method and the like.

The polyimide according to the embodiment of the present invention is a reaction product of a reactive component (α) (hereinafter referred to as (α) component) comprising the diamine compound (a1) according to the embodiment of the present invention (hereinafter referred to as (a1) component) and tetracarboxylic dianhydride (a2) (hereinafter referred to as (a2) component). That is, the (α) component comprises a monomer unit derived from the diamine compound (a1) and a monomer unit derived from the tetracarboxylic dianhydride (a2).

Various known tetracarboxylic dianhydrides can be used as the (a2) component without particular limitations. Specifically, examples of the (a2) component include an aliphatic tetracarboxylic dianhydride, an aromatic tetracarboxylic dianhydride and the like.

Examples of the aliphatic tetracarboxylic dianhydride include 1,2,3,4-butane tetracarboxylic dianhydride, 1,2,3,4-cyclobutane tetracarboxylic dianhydride, 1,2,3,4-cyclopentane tetracarboxylic dianhydride, 1,2,4,5-cyclohexanetetracarboxylic dianhydride, bicyclo(2,2,2)oct-7-ene-2,3,5,6 tetracarboxylic dianhydride, bicyclo(2,2,2)octane-2,3,5,6-tetracarboxylic dianhydride, 2,3,5-tricarboxycyclopentylacetic dianhydride, 3,5,6tricarboxynorbornan-2-acetic dianhydride, 4,5-tetrahydrofurantetracarboxylic dianhydride, 3-carboxymethyl-1,2,4-cyclopentane tricarboxylic acid-1,4:2,3 dianhydride and the like. The aliphatic tetracarboxylic dianhydrides may be used in combination.

Examples of the aromatic tetracarboxylic dianhydrides include pyromellitic dianhydride, 4,4'-oxydiphthalic dianhydride, 3,3',4,4'-benzophenonetetracarboxylic dianhydride, 3,3',4,4'-diphenyl ether tetracarboxylic dianhydride, 3,3',4,4'-diphenylsulfone tetracarboxylic dianhydride, 1,2,3,4-benzenetetracarboxylic dianhydride, 1,4,5,8-naphthalenetetracarboxylic dianhydride, 2,3,6,7-naphthalenetetracarboxylic dianhydride, 3,3',4,4'-biphenyltetracarboxylic dianhydride, 2,2',3,3'-biphenyltetracarboxylic dianhydride, 2,3,3',4'-biphenyltetracarboxylic dianhydride, 2,3,3',4'-benzophenonetetracarboxylic dianhydride, 2,3,3',4'-diphenylether tetracarboxylic dianhydride, 2,3,3',4'-diphenylsulfone tetracarboxylic dianhydride, 2,2-bis(3,3',4,4'-tetracarboxyphenyl)tetrafluoropropane dianhydride, 2,2'-bis(3,4-dicarboxyphenoxyphenyl) sulfone dianhydride, 2,2-bis(2,3-dicarboxyphenyl) propane dianhydride, 2,2-bis(3,4-dicarboxyphenyl) propane dianhydride, 4,4'-[propane-2,2-diylbis(1,4-phenyleneoxy)] diphthalic dianhydride and the like. The aromatic tetracarboxylic dianhydrides may be used in combination.

Various known diaminopolysiloxanes (a3) (hereinafter, (a3) component) may be included in the (α) component. Specifically, examples of the (a3) component include α,ω-bis(2-aminoethyl) polydimethylsiloxane, α,ω-bis(3-aminopropyl) polydimethylsiloxane, α,ω-bis(4-aminobutyl) polydimethylsiloxane, α,ω-bis(5-aminopentyl) polydimethylsiloxane, α,ω-bis[3-(2-aminophenyl)propyl] polydimethylsiloxane, α,ω-bis[3-(4-aminophenyl)propyl] polydimethylsiloxane and the like. The (a3) components may be used in combination.

The (α) component may comprise optionally diamine (hereinafter referred to as (a4) component) other than the (a1) component and the (a3) component. Specifically, examples of the (a4) component include alicyclic diamines such as diaminocyclohexane, diaminodicyclohexylmethane, dimethyl-diaminodicyclohexylmethane, tetramethyl-diaminodicyclohexylmethane, diaminodicyclohexylpropane, diaminobicyclo[2.2.1]heptane, bis(aminomethyl)-bicyclo[2.2.1]heptane, 3(4),8(9)-bis(aminomethyl)tricyclo[5.2.1.02,6]decane, 1,3-bis-aminomethyl cyclohexane and isophorone diamine; bis-aminophenoxyphenylpropanes such as 2,2-bis[4-(3-aminophenoxy)phenyl] propane and 2,2-bis[4-(4-aminophenoxy)phenyl] propane; diaminodiphenyl ethers such as 3,3'-diaminodiphenyl ether, 3,4'-diaminodiphenyl ether and 4,4'-diaminodiphenyl ether; phenylenediamines such as p-phenylenediamine and m-phenylenediamine; diaminodiphenyl sulfides such as 3,3'-diaminodiphenyl sulfide, 3,4'-diaminodiphenyl sulfide and 4,4'-diaminodiphenyl sulfide; diamino diphenyl sulfones such as 3,3'-diaminodiphenyl sulfone, 3,4'-diaminodiphenyl sulfone and 4,4'-diaminodiphenyl sulfone; diaminobenzophenones such as 3,3'-diaminobenzophenone, 4,4'-diaminobenzophenone and 3,4'-diaminobenzophenone; diaminodiphenylmethanes such as 3,3'-diaminodiphenylmethane, 4,4'-diaminodiphenylmethane and 3,4'-diaminodiphenylmethane; diaminophenyl propanes such as 2,2-di(3-aminophenyl)propane, 2,2-di(4-aminophenyl)propane and 2-(3-aminophenyl)-2-(4-aminophenyl)propane; diaminophenyl hexafluoropropanes such as 2,2-di(3-aminophenyl)-1,1,1,3,3,3-hexafluoropropane, 2,2-di(4-aminophenyl)-1,1,1,3,3,3-hexafluoropropane and 2-(3-aminophenyl)-2-(4-aminophenyl)-1, 1, 1,3,3,3-hexafluoropropa ne; diaminophenyl phenylethanes such as 1,1-di(3-aminophenyl)-1-phenylethane, 1,1-di(4-aminophenyl)-1-phenylethane and 1-(3-aminophenyl)-1-(4-aminophenyl)-1-phenylethane; bisaminophenoxy benzenes such as 1,3-bis(3-aminophenoxy) benzene, 1,3-bis(4-aminophenoxy) benzene, 1,4-bis(3-aminophenoxy) benzene and 1,4-bis(4-aminophenoxy) benzene; bisaminobenzoyl benzenes such as 1,3-bis(3-aminobenzoyl) benzene , 1,3-bis(4-aminobenzoyl) benzene, 1,4-bis(3-aminobenzoyl) benzene and 1,4-bis(4-aminobenzoyl) benzene; bisaminodimethylbenzenes such as 1,3-bis(3-amino-α,α-dimethylbenzyl) benzene, 1,3-bis(4-amino-α,α-dimethylbenzyl) benzene 1,4-bis(3-amino-α,α-dimethylbenzyl) benzene and 1,4-bis(4-amino-α,α-dimethylbenzyl) benzene; bisaminoditrifluoromethylbenzyl benzenes such as 1,3-bis(3-amino-α,α-ditrifluoromethylbenzyl) benzene, 1,3-bis(4-amino-α,α-ditrifluoromethylbenzyl) benzene, 1,4-bis(3-amino-α,α-ditrifluoromethylbenzyl) benzene and 1,4-bis(4-amino-α,α-ditrifluoromethylbenzyl) benzene; aminophenoxy biphenyls such as 2,6-bis(3-aminophenoxy) benzonitrile, 2,6-bis(3-aminophenoxy) pyridine, 4,4'-bis(3-aminophenoxy) biphenyl and 4,4'-bis(4-aminophenoxy) biphenyl; amino-phenoxyphenyl ketones such as bis[4-(3-aminophenoxy)phenyl] ketone and bis[4-(4-aminophenoxy)phenyl] ketone; aminophenoxy phenyl sulfides such as bis[4-(3-aminophenoxy)phenyl] sulfide and bis[4-(4-aminophenoxy)phenyl] sulfide; amino-phenoxyphenyl sulfones such as bis[4-(3-aminophenoxy)phenyl] sulfone and bis[4-(4-aminophenoxy)phenyl] sulfone; amino-phenoxyphenyl ethers such as bis[4-(3-aminophenoxy)phenyl] ether and bis[4-(4-aminophenoxy)phenyl] ether; aminophenoxyphenylpropanes such as 2,2-bis[4-(3-aminophenoxy)phenyl] propane, 2,2-bis[3-(3-aminophenoxy)phenyl]-1,1,1,3,3,3-hexafluoropropane and 2,2-bis[4-(4-aminophenoxy)phenyl]-1,1,1,3,3,3-hexafluoropropane; and others such as 1,3-bis[4-(3-aminophenoxy)benzoyl] benzene, 1,3-bis[4-(4-aminophenoxy)benzoyl] benzene, 1,4-bis[4-(3-aminophenoxy)benzoyl] benzene, 1,4-bis[4-(4-aminophenoxy)benzoyl] benzene, 1,3-bis[4-(3-aminophenoxy)-α,α-dimethylbenzyl] benzene, 1,3-bis[4-(4-aminophenoxy)-α,α-dimethylbenzyl] benzene, 1,4-bis[4-(3-aminophenoxy)-α,α-dimethylbenzyl] benzene, 1,4-bis[4-(4-aminophenoxy)-α,α-dimethylbenzyl] benzene, 4,4'-bis[4-(4-aminophenoxy)benzoyl] diphenylether, 4,4'-bis[4-(4-amino-α,α-dimethylbenzyl)phenoxy] benzophenone, 4,4'-bis[4-(4-amino-α,α-dimethylbenzyl)phenoxy] diphenyl sulfone, 4,4'-bis[4-(4-aminophenoxy)phenoxy] diphenyl sulfone, 3,3'-diamino-4,4'-phenoxy-benzophenone, 3,3'-diamino-4,4'-dibiphenoxy-benzophenone, 3,3'-diamino-4-phenoxy-benzophenon, 3,3'-diamino-4-biphenoxy benzophenone, 6,6'-bis(3-aminophenoxy) 3,3,3',3,'-tetramethyl-1,1'-spirobiindan 6,6'-bis(4-aminophenoxy) 3,3,3',3',-tetramethyl-1,1'-spirobiindan, 1,3-bis(3-aminopropyl) tetramethyldisiloxane, 1,3-bis(4-aminobutyl) tetramethyldisiloxane, bis(aminomethyl) ether, bis(2-aminoethyl) ether, bis(3-aminopropyl) ether, bis(2-aminomethoxy)ethyl]-ether, bis[2-(2-aminoethoxy)ethyl] ether, bis[2-(3-aminoprotoxy)ethyl] ether, 1,2-bis(aminomethoxy) ethane, 1,2-bis(2-aminoethoxy) ethane, 1,2-bis[2-(aminomethoxy)ethoxy] ethane, 1,2-bis[2-(2-aminoethoxy)ethoxy] ethane, ethylene glycol bis(3-aminopropyl) ether, diethylene glycol bis(3-aminopropyl) ether, triethylene glycol bis(3-aminopropyl) ether, ethylenediamine, 1,3-diaminopropane, 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, 1,12-diaminododecane and the like. The (a4) components may be used in combination.

A molar ratio of the (a2) component as an acid component to the (a1) component, the (a3) component and the (a4) component as a diamine component, that is, [(a2)/[(a1)+(a3)+(a4)]] is not particularly limited. From the viewpoint of heat resistance, the molar ratio [(a2)/[(a1)+(a3)+(a4)]] is usually approximately from 1 to 1.5, preferably from 1 to 1.2.

In the diamine component, proportions of the (a1) component, (a3) component and (a4) component are not particularly limited. From the viewpoint of heat resistance, the proportions are usually as follows.
(a1) component: from about 10 mol% to about 100 mol%, preferably from about 30 mol% to about 100 mol%
(a3) component: from about 50 mol% to 0 mol%, preferably from about 5 mol% to 0 mol%
(a4) component: from about 90 mol% to 0 mol%, preferably from about 70 mol% to 0 mol%

The polyimide of the embodiment of the present invention can be produced by various known methods. Specifically, for example, (a1) component and (a2) component, and optionally the (a3) component and/or (a4) component are subjected to polyaddition reaction in the presence of an organic solvent at a temperature of from a room temperature (25°C) to about 120°C, preferably at a temperature of from a room temperature (25°C) to about 80°C for 1 hour to about 24 hours to synthesize a polyamic acid solution. Then, the obtained polyamic acid solution may be further subjected to imidization reaction (dehydration ring-closing reaction) as it is at a temperature of from about 80°C to about 250°C, preferably at a temperature of from about 100°C to about 200°C for about 0.5 hour to about 50 hours. Or, after casting or coating the polyamic acid solution on a support such as a glass plate, a metal plate (e.g., SUS) or a plastic film (e.g., a polyethylene terephthalate film, a polyimide film), heating treatment may be carried out at a temperature of from about 80°C to about 300°C, preferably from about 150°C to about 300°C for about 0.5 hour to 50 hours for the imidization reaction.

Examples of the organic solvents include amide solvents such as N-methyl-2-pyrrolidone, N,N-dimethylacetamide, N,N-diethylacetamide and N,N-dimethylformamide; a lactone solvent such as γ-butyrolactone; a ketone solvent such as methyl isobutyl ketone; ether solvents such as ethylene glycol dimethyl ether, diethylene glycol dimethyl ether and dipropylene glycol dimethyl ether; alicyclic solvents such as cyclohexane and methylcyclohexanone; alcohol solvents such as methanol, ethanol, propanol, benzyl alcohol and cresol; aromatic solvents such as toluene and xylene; and the like. The organic solvents may be used in combination. Among these, aprotic polar solvents are preferable as the organic solvent.

During the above-mentioned imidation reaction, various known catalysts can be used. Specifically, examples of the catalyst include aliphatic tertiary amines such as triethylamine; aromatic tertiary amines such as dimethylaniline; heterocyclic tertiary amines such as pyridine, picoline and isoquinoline; and the like. The catalysts may be used in combination.

During the above-mentioned imidation reaction, various known dehydrating agents can be used. Specifically, examples of the dehydrating agent include aliphatic acid anhydrides such as acetic anhydride, aromatic acid anhydrides such as benzoic anhydride and the like. The dehydrating agents may be used in combination.

For the identification of the polyimide of the embodiment of the present invention, various known means can be adopted. Specifically, examples of the identification means of polyimide include infrared spectroscopy, nuclear magnetic resonance, gel permeation chromatography (GPC) and the like. Physical properties of the polyimide of the embodiment of the present invention is not particularly limited. From the viewpoint of heat resistance, in the physical properties of the polyimide, a weight-average molecular weight (a value converted with polystyrene by the gel permeation chromatography (GPC)) is usually from about 5,000 to about 50,000.

As mentioned above, the diamine compound of the embodiment of the present invention has a structure of the resin acid dimer residues having a rigid and bulky skeleton in the molecule thereof. Therefore, the diamine compound can be suitably used for a monomer of an engineering plastic having heat resistance and abrasion resistance. For example, it can be considered that the diamine compound according to the present embodiment is useful as a monomer of the polyimide in applications where heat resistance and mechanical properties are required. Specifically, the diamine compound according to the present embodiment is expected as a starting material of polyimide to be utilized in applications such as parts for aerospace industry (such as a satellite), automotive parts, semiconductor material (resist, etc.), military, aircraft, electric and electronic parts, heat-resistant fiber, industrial equipment and the like. Further, it can be considered that the polyimide is useful for applications other than those mentioned above where heat resistance and mechanical properties are required.

One embodiment of the present invention is as described above. The present invention is not limited to the above embodiment. The embodiments described above is an embodiment of the invention having the following configuration.
(1) Diamine compound represented by H₂NH₂C-Ro²-CH₂NH₂ (wherein, Ro² denotes a resin acid dimer residue.).
   According to such a configuration, the diamine compound is more excellent in heat resistance than conventional diamine compounds. Therefore, the diamine compound may be developed in a variety of new applications.
(2) Diamine compound described in (1) above having the following structure. 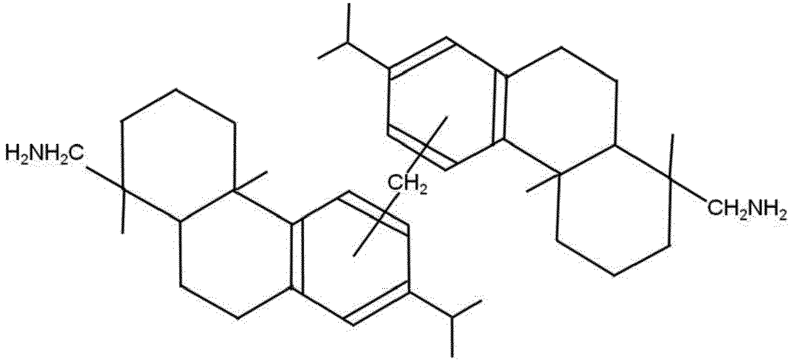
   According to such a configuration, it is easy to synthesize the polyimide compound using the diamine compound of the above structure. Therefore, such a diamine compound is further developed easily for a variety of new applications.
(3) Method of preparing a diamine compound comprising a step of preparing the diamine compound described in (1) or (2) above by subjecting the dinitrile compound represented by N≡C-Ro²-C≡N (wherein, Ro² denotes a resin acid dimer residue) to reduction reaction.
   According to such a configuration, in the above-mentioned method of preparing the diamine compound, the diamine compound is obtained in high yield compared with the method of preparing a diamine compound by subjecting a monoamine represented by Ro¹-CH₂NH₂ (wherein Ro¹ denotes a resin acid residue) to dimerization reaction.
(4) Method of preparing a diamine compound described in (3) above, further comprising a step of preparing the dinitrile compound by subjecting a mononitrile compound represented by Ro¹-C≡N (wherein Ro¹ denotes a resin acid residue) to dimerization reaction.
   According to such a configuration, the preparation process of the diamine compound is shortened since it is possible to dispensed with a step of preparing the monoester compound represented by Ro¹-COO-R.
(5) Method of preparing a diamine compound described in (3) above, further comprising a step of preparing the dinitrile compound by reacting a diester compound represented by R-OOC-Ro²-COO-R (wherein Ro² represents a resin acid dimer residue, and R represents an alkyl group having 1 to 10 carbon atoms) with ammonia.
   According to such a configuration, since the preparation method of the diamine compound uses a high purity diester compound as a starting material, refining of the obtained diamine compound is facilitated
(6) Polyimide including a monomer unit derived from the diamine compound described in (1) or (2) above and a monomer unit derived from a tetracarboxylic dianhydride.
   According to such a configuration, the obtained polyimide is useful in applications where heat resistance and abrasion resistance are required because it includes the monomer unit derived from the diamine compound.

### EXAMPLE

The present invention will be explained below in detail by way of Examples. The present invention is not limited thereto.

### Preparation Example 1 (Preparation of dimerized rosin diester)

Into a reaction vessel equipped with a thermometer, a stirrer, a nitrogen introduction tube and a pressure reducing device were charged 100 g of unpurified rosin (China gum rosin) having an acid value of 170.0 mgKOH/g, 100 g of xylene and 83.3g of methanol. While stirring a mixture, 46.4 g of thionyl chloride was added to the mixture dropwise over 2 hours for methyl esterification of the unpurified rosin. After completion of the dropwise addition, the mixture was subjected to and then heating and stirring at a reflux temperature for 5 hours, dispensed with xylene and methanol were distilled off under the conditions of a temperature lower than 200°C and a reduced pressure of 1,300 Pa, followed by further distillation under a liquid temperature of 230°C and a reduced pressure of 1,200 Pa to obtain 70 g of a purified rosin methyl ester. An acid value was 0.5 mgKOH/g (unreacted ratio 0.3%).

Then, 900 g of the obtained purified rosin methyl ester, 900 g of xylene, 45.0 g of zinc chloride and 45.0 g of sulfuric acid were charged in a similar reaction vessel, followed by polymerization reaction for 6 hours at 145°C under nitrogen stream. After the xylene solution of the reaction product was washed by adding 7 g of concentrated hydrochloric acid and 500 g of warm water thereto, further washing was carried out twice with 500 g each of warm water. Xylene was distilled off from the xylene solution after the washing under the conditions of a liquid temperature lower than 200°C and a reduced pressure of 6,000 Pa, and then a decomposed product of the purified rosin methyl ester and an unreacted purified rosin methyl ester were distilled off under a liquid temperature of 275°C and a reduced pressure of 150 Pa to obtain 405 g of a dimerized rosin diester (polymerized rosin ester).

### Preparation Example 2 (Preparation of dimerized rosin dinitrile from dimerized rosin diester)

Into a reaction vessel equipped with a thermometer, a stirrer and a nitrogen introducing tube were charged 300 g of the dimerized rosin diester obtained in Preparation Example 1 and 10 g of zinc oxide. Then, 700 mL/min of ammonia gas was continuously supplied, followed by 40-hour reaction at 290°C under nitrogen atmosphere to obtain 250 g of dimerized rosin dinitrile (polymerized rosin nitrile).

### Preparation Example 3 (Preparation of rosin nitrile)

Into a reaction vessel equipped with a thermometer, a stirrer, a nitrogen introduction tube and a pressure reducing device were charged 300 g of unpurified rosin (China gum rosin) having an acid value of 172.3 mgKOH/g and 10 g of zinc oxide. Then, 700 mL/min of ammonia gas was continuously supplied, followed by 16-hour reaction at 260°C under nitrogen atmosphere. After the reaction, distillation was carried out under the conditions of a liquid temperature lower than 250°C and a reduced pressure of 1300 Pa to obtain 210 g of a purified rosin nitrile. An acid value was 0.3 mgKOH/g (unreacted ratio 0.2%).

### Preparation Example 4 (Preparation of dimerized rosin dinitrile from rosin nitrile)

After charging 100 g of the purified rosin nitrile obtained in Preparation Example 3, 20 g of xylene and 7.0 g of methanesulfonic acid, a polymerization reaction was carried out at 140°C for 5 hours under a nitrogen stream. After washing by adding 20 g of warm water to a xylene solution of the reaction product, further washing was carried out 4 times with 60 g each of warm water. After the washing, xylene was distilled off from the xylene solution under the conditions of a liquid temperature lower than 200°C and a reduced pressure of 6,000 Pa, and then a decomposed product of the purified rosin nitrile and unreacted purified rosin nitrile were further distilled under a liquid temperature of 270°C and a reduced pressure of 150 Pa to obtain 40 g of dimerized rosin dinitrile (polymerized rosin nitrile).

### Preparation Example 5 (Preparation of dimerized rosin diester)

Into a reaction vessel equipped with a thermometer, a stirrer, a nitrogen introduction tube and a pressure reducing device were charged 100 g of purified disproportionated rosin (trade name "KR-614" manufactured by Arakawa Chemical Industries, Ltd.) having an acid value of 170.0 mgKOH/g, 100 g of xylene and 83.3 g of methanol. While stirring a mixture, 46.4 g of thionyl chloride was added to the mixture dropwise over 2 hours for methyl esterification of the purified disproportionated rosin. After completion of the dropwise addition and then heating and stirring at a reflux temperature for 5 hours, xylene and methanol were distilled off under the conditions of a liquid temperature lower than 200°C and a reduced pressure of 1,300 Pa, followed by further distillation under a liquid temperature of 230°C and a reduced pressure of 1,200 Pa to obtain 70 g of a purified disproportionated rosin methyl ester. An acid value was 0.5 mgKOH/g (unreacted ratio 0.3%). According to GC measurement, a neutral ingredient amount in the purified rosin methyl ester was 7.5% (rosin ester purity 92.2%), and a total amount of dehydroabietic acid was 71.5%.

Then, 100 mL of ethyl acetate was poured into a reaction vessel equipped with a thermometer, a stirrer and a nitrogen introduction tube, and 50 mL of sulfuric acid was added thereto dropwise at 15° to 25°C with cooling. Subsequently, 100.0 g of the obtained purified disproportionated rosin methyl ester and 7.1 g of paraformaldehyde were added and stirred at 40°C for 4 hours. To the reaction solution were added 200 mL of ice water and 200 mL of ethyl acetate to separate an aqueous phase and an organic phase was repeatedly washed with water. After the organic phase was dried with anhydrous magnesium sulfate, ethyl acetate was distilled off under reduced pressure, and 100 mL of methanol was added to a residue, followed by stirring at room temperature for 1 hour. After filtering off solidified crystals and washing with methanol, 70 g of white crystals of dimerized rosin diester (methylenebis dehydroabietic acid dimethyl) was obtained by performing recrystallization twice from ethyl acetate/methanol.

### Preparation Example 6 (Preparation of dimerized rosin dinitrile from dimerized rosin diester)

Into a reaction vessel equipped with a thermometer, a stirrer and a nitrogen introducing tube were charged 300 g of the dimerized rosin diester (methylenebis dehydroabietic acid dimethyl) obtained in Preparation Example 5 and 10 g of zinc oxide. Then, 700 mL/min of ammonia gas was continuously supplied, followed by 18-hour reaction at 290°C under nitrogen atmosphere to obtain 250 g of dimerized rosin dinitrile (methylenebis dehydroabietyl dinitrile).

### Example 1

Into a reaction vessel equipped with a thermometer, a stirrer and a nitrogen introducing tube were charged 10 g of dimerized rosin dinitrile (polymerized rosin nitrile) obtained in Preparation Example 2, 40 g of toluene and 30 g of a 70% toluene solution of SMEAH (sodium bis(2-methoxyethoxy)aluminum hydride), followed by reaction at 110°C for 3 hours under a nitrogen stream. After completion of the reaction, 100 g of THF and 30 g of water were added and 30 g of a 15% aqueous solution of NaOH was added to treat an unreacted reducing agent. Thereafter, washing was carried out 5 times with 50 g each of water, and toluene was removed under conditions of a temperature lower than 120°C and a reduced pressure of 1,300 Pa to obtain 10 g of dimerized rosin diamine.

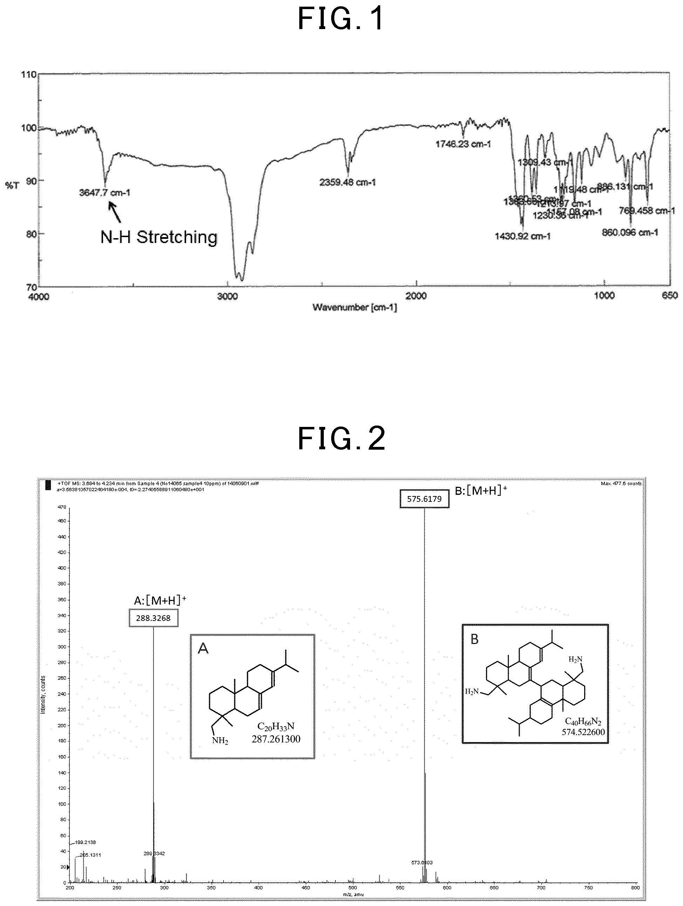

An infrared absorption spectrum chart of the dimerized rosin diamine obtained in Example 1 is shown in FIG. 1. The measurement was carried out using commercially available equipment (product name "FT/IR 4100" manufactured by JASCO Corporation). In the spectrum chart, disappearance of a peak derived from C≡N near 2,230 cm⁻¹ and appearance of a peak derived from C-NH₂ near 3,400 cm⁻¹ were observed.

A DI/MS chart in the dimerized rosin diamine obtained in Example 1 is shown in FIG. 2. The measurement was carried out using commercially available equipment (QSTAR XL Hybrid LC/MS/MS system, Kabushiki Kaisha AB Sciex). A signal A of [M+H]⁺=288 corresponds to a molecular weight of rosin amine of a monomer (a substance described as A in FIG. 2), and a signal B of [M+H]⁺=576 corresponds to a molecular weight of a polymerized rosin amine (a substance described as B in FIG. 2). Therefore, it is seen that the dimerized rosin diamine comprises rosin amine of a monoamine compound and polymerized rosin amine of a diamine compound.

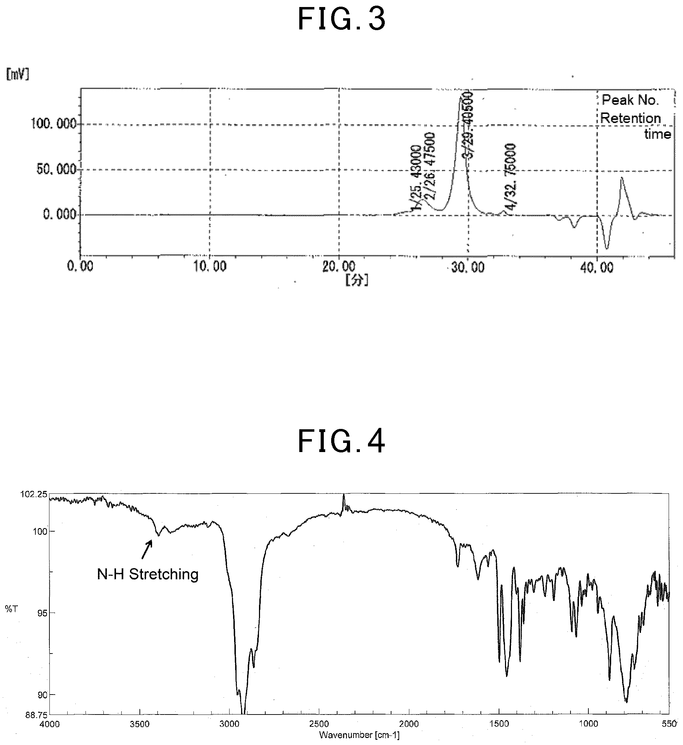

A GPC chart showing a component ratio of the diamine compound in the dimerized rosin diamine obtained in Example 1 is shown in FIG. 3. Incidentally, the chart shows the result obtained using a commercially available measuring apparatus (HLC-8220 manufactured by Tosoh Corporation) and a column (Tskgel column manufactured by Tosoh Corporation) (developing solvent THF, feeding rate 1.0 mL/min). From the chart, a purity of the diamine compound was 81.2 wt%. Incidentally, the diamine compound is a diamine compound of the above-mentioned structure (I).

### Example 2

Into a reaction vessel equipped with a thermometer, a stirrer and a nitrogen introducing tube were charged 20 g of dimerized rosin dinitrile (polymerized rosin nitrile) obtained in Preparation Example 4, 80 g of toluene and 60 g of a 70% toluene solution of SMEAH (sodium bis(2-methoxyethoxy)aluminum hydride), followed by reaction at 110°C for 3 hours under a nitrogen stream. After completion of the reaction, 200 g of THF and 60 g of water were added and 60 g of a 15% aqueous solution of NaOH was added to treat an unreacted reducing agent. Thereafter, washing was carried out 5 times with 100 g each of water, and toluene was removed under conditions of a temperature lower than 120°C and a reduced pressure of 1,300 Pa to obtain 20 g of dimerized rosin diamine.

### Example 3

Into a reaction vessel equipped with a thermometer, a stirrer and a nitrogen introducing tube were charged 10 g of dimerized rosin dinitrile (methylenebis dehydroabietyl dinitrile) obtained in Preparation Example 6, 40 g of toluene and 30 g of a 70% toluene solution of SMEAH (sodium bis(2-methoxyethoxy)aluminum hydride) and reaction was conducted at 110°C for 3 hours under a nitrogen stream. After completion of the reaction, 100 g of THF and 30 g of water were added and 30 g of a 15% aqueous solution of NaOH was added to treat an unreacted reducing agent. Thereafter, washing was carried out 5 times with 50 g each of water, and toluene was removed under conditions of a temperature lower than 120°C and a reduced pressure of 1,300 Pa to obtain 10 g of dimerized rosin diamine.

An infrared absorption spectrum chart of the dimerized rosin diamine obtained in Example 3 is shown in FIG. 4. The measurement was carried out using commercially available equipment (product name "FT/IR 4100" manufactured by JASCO Corporation). In the spectrum chart, disappearance of a peak derived from C≡N near 2,230 cm⁻¹ and appearance of a peak derived from C-NH₂ near 3,400 cm⁻¹ were observed.

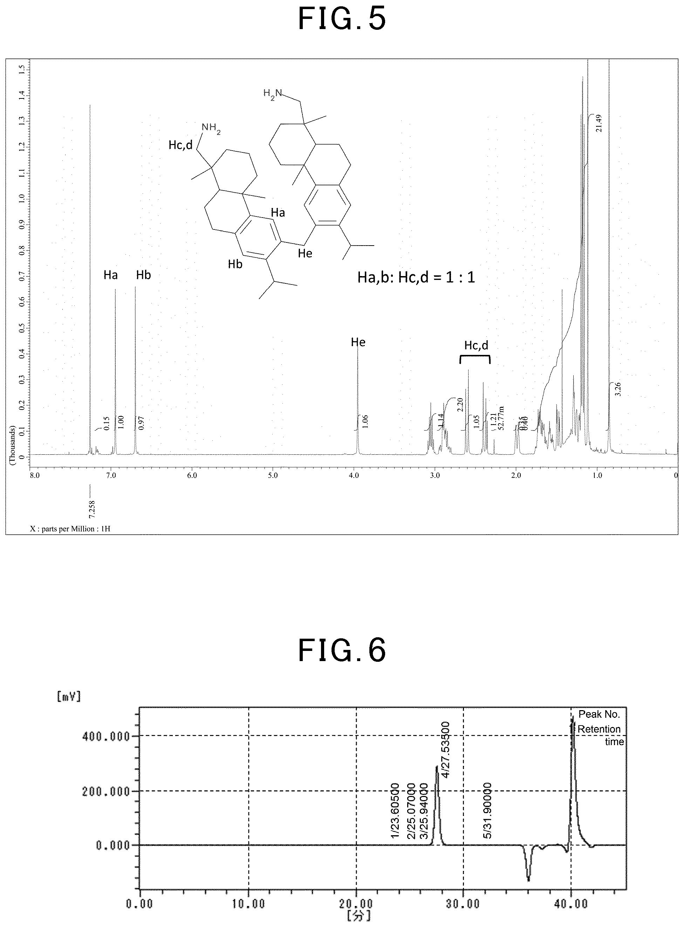

A ¹H-NMR chart of the dimerized rosin diamine obtained in Example 3 is shown in FIG. 5. The measurement was carried out using Agilent 400-MR, manufactured by Agilent Technologies Japan, Ltd. Hc and Hd is doublet 1:1 and therefore, it is seen that nitrile disappeared and was reduced to amine. Further, aromatic protons Ha and Hb are singlet 1:1, and a singlet of He is seen in a low magnetic field near δ4.0. Therefore, it is seen that the dimerized rosin diamine was methylenebis dehydroabietyl diamine of the diamine compound described in FIG. 5. Further, the diamine compound is a diamine compound of the above-mentioned structure (II).

A GPC chart showing a component ratio of the diamine compound in the dimerized rosin diamine obtained in Example 3 is shown in FIG. 6. Incidentally, the chart shows the result obtained using a commercially available measuring apparatus (HLC-8220 manufactured by Tosoh Corporation) and a column (Tskgel column manufactured by Tosoh Corporation) (developing solvent THF, feeding rate 1.0 mL/min). From the chart, a purity of the diamine compound was 99.4 wt%.

### Example 4 (Preparation of polyimide)

Into a reaction vessel equipped with a stirrer, a water separator, a thermometer and a nitrogen introducing tube were charged 1.46 g of dimerized rosin diamine (diamine compound 99.4 wt%) obtained in Example 3 and 9.7 g of N-methylpyrrolidone, followed by stirring at room temperature under a nitrogen stream for dissolution thereof. Then, 0.78 g of 4,4'-oxydiphthalic dianhydride (available from Tokyo Chemical Industry Co., Ltd.) was added, followed by 24-hour reaction at a room temperature under a nitrogen gas stream with stirring to prepare a polyamic acid solution (non-volatile matter 19.0%). Then, 2.0 g of the polyamic acid solution was coated on a PET film (trade name "Lumirror 75-T60" available from PANAC Corporation) by a film applicator (trade name "4-sided film applicator" available from All Good Company), followed by drying at 120°C for 30 minutes by a fair wind dryer. After the drying, the obtained polyamic acid film was peeled off from the PET film, fixed to a SUS metal plate of 95 mm long × 55 mm wide × 10 mm thick, and heated at 200°C for 1 hour, and further heated at 250°C for 1 hour to prepare polyimide.

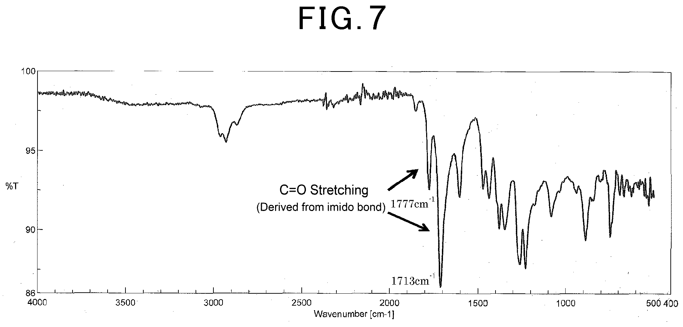

An infrared absorption spectrum chart of the polyimide obtained in Example 4 is shown in FIG. 7. The measurement was carried out using commercially available equipment (product name "FT/IR 4100" manufactured by JASCO Corporation). In the spectrum chart, disappearance of a peak derived from N-H of an amide bond near 3,314 cm⁻¹ and a peak derived from C-O of an amide bond near 1,645 cm⁻¹ was confirmed and appearance of a peak derived from C=O of a cyclic imide bond near 1,713 cm⁻¹ and 1,777 cm⁻¹ was observed.

### Reference Example 1 (Preparation of polyimide)

Into a reaction vessel equipped with a stirrer, a water separator, a thermometer and a nitrogen introducing tube were charged 1.45 g of dimerized rosin diamine (diamine compound 81.2 wt%) obtained in Example 1 and 9.7 g of N-methylpyrrolidone, followed by stirring at room temperature under a nitrogen stream for dissolution thereof. Then, 0.78 g of 4,4'-oxydiphthalic dianhydride (available from Tokyo Chemical Industry Co., Ltd.) was added, followed by 24-hour reaction at a room temperature under a nitrogen gas stream with stirring to prepare a polyamic acid solution (non-volatile matter 19.0%). Then, 2.0 g of the polyamic acid solution was coated on a PET film (trade name "Lumirror 75-T60" available from PANAC Corporation) by a film applicator (trade name "4-sided film applicator" available from All Good Company), followed by drying at 120°C for 30 minutes by a fair wind dryer. After the drying, the obtained polyamic acid film was peeled off from the PET film, fixed to a SUS metal plate of 95 mm long × 55 mm wide × 10 mm thick, and heated at 200°C for 1 hour, and further heated at 250°C for 1 hour to prepare polyimide. However, because the obtained polyimide was fragile and could not be formed into a film. It is conjectured that this is because the dimerized rosin amine obtained in Example 1 contained relatively a large amount of not only a diamine compound but also a monoamine compound, a molecular weight of the obtained polyimide became smaller.

From the results of Example 4 and Reference Example 1, it was found that among the diamine compounds of the present invention, preparation of polyimide particularly with the diamine compound represented by the structure (II) is easier than preparation of polyimide with the diamine compounds of structure (I).

## Claims

1. A diamine compound represented by H₂NH₂C-Ro²-CH₂NH₂, wherein Ro² represents a residue of a resin acid dimer.

2. The diamine compound of claim 1, having the following structure.

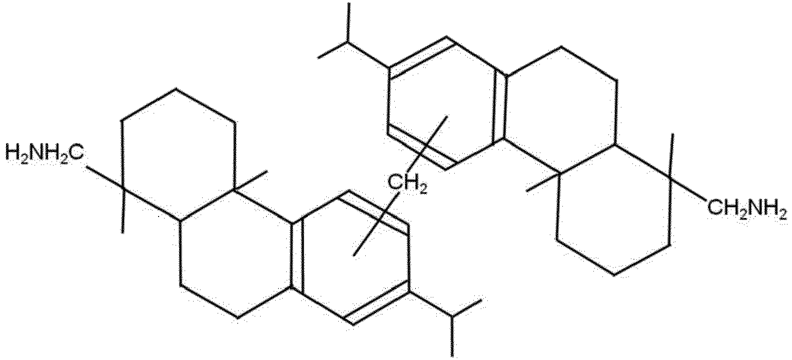

3. A method of preparing a diamine compound, the method comprising a step of preparing the diamine compound of claim 1 or 2 by subjecting a dinitrile compound represented by N≡C-Ro²-C≡N, wherein Ro² represents a residue of a resin acid dimer, to reduction reaction.

4. The method of preparing a diamine compound of claim 3, the method further comprising a step of preparing the dinitrile compound by subjecting a mononitrile compound represented by Ro¹-C≡N, wherein Ro¹ represents a residue of a resin acid, to dimerization reaction.

5. The method of preparing a diamine compound of claim 3, the method further comprising a step of preparing the dinitrile compound by allowing a diester compound represented by R-OOC-Ro²-COO-R, wherein Ro² represents a residue of a resin acid dimer and R represents an alkyl group having 1 to 10 carbon atoms, to react with ammonia.

6. Polyimide comprising a monomer unit derived from the diamine compound of claim 1 or 2 and a monomer unit derived from tetracarboxylic dianhydride.
